# EUROPEAN PATENT SPECIFICATION

(11) **EP 2 240 189 B1**
(45) Date of publication and mention of the grant of the patent: **30.12.2015**
(21) Application number: 09700504.5
(22) Date of filing: 02.01.2009
(51) Int. Cl.: A61K 35/14, A61K 35/28, A61P 25/02, A61P 25/28, A61K 8/98, A61Q 19/00

(54) **MICROVESICLES**
MIKROVESIKEL
MICROVÉSICULES

(30) Priority: 04.01.2008 GB 0800107; 17.03.2008 GB 0804932
(43) Date of publication of application: 20.10.2010
(73) Proprietor: Lydac Neuroscience Limited, Bath BA1 5JR (GB)
(72) Inventor: RAY, Stephen, Bath BA1 5JR (GB)
(74) Representative: Greenwood, Matthew David
(86) International application number: PCT/GB2009/000004
(87) International publication number: WO 2009/087361

(56) References cited:
- WO-A-2005/121369
- RATAJCZAK J ET AL: "Embryonic stem cell-derived microvesicles reprogram hematopoietic progenitors: evidence for horizontal transfer of mRNA and protein delivery" LEUKEMIA, MACMILLAN PRESS LTD, US, vol. 20, no. 5, 1 May 2006 (2006-05-01), pages 847-856, XP002456317 ISSN: 0887-6924 cited in the application
- ALIOTTA JASON M ET AL: "Alteration of marrow cell gene expression, protein production, and engraftment into lung by lung-derived microvesicles: A novel mechanism for phenotype modulation" STEM CELLS (MIAMISBURG), vol. 25, no. 9, September 2007 (2007-09), pages 2245-2256, XP002523307 ISSN: 1066-5099 cited in the application
- DATABASE BIOSIS [Online] BIOSCIENCES INFORMATION SERVICE, PHILADELPHIA, PA, US; 1995, SOROKOVOI V I ET AL: "Erythropoiesis stimulation in bleeding during pregnancy by means of microvesicles made of erythrocyte plasmalemma" XP002523309 Database accession no. PREV199598503793 & ARKHIV PATOLOGII, vol. 57, no. 2, 1995, pages 65-68, ISSN: 0004-1955
- DEREGIBUS MARIA CHIARA ET AL: "Endothelial progenitor cell derived microvesicles activate an angiogenic program in endothelial cells by a horizontal transfer of mRNA" BLOOD, AMERICAN SOCIETY OF HEMATOLOGY, US, vol. 110, no. 7, 1 October 2007 (2007-10-01), pages 2440-2448, XP009101333 ISSN: 0006-4971 cited in the application
- VALENTI ROBERTA ET AL: "Tumor-released microvesicles as vehicles of immunosuppression" CANCER RESEARCH, vol. 67, no. 7, April 2007 (2007-04), pages 2912-2915, XP002523308 ISSN: 0008-5472
- HUGEL BENEDICTE ET AL: "Membrane microparticles: Two sides of the coin" PHYSIOLOGY (BETHESDA), vol. 20, no. February, February 2005 (2005-02), pages 22-27, XP009115152 ISSN: 1548-9213
- NAKAMURA ET AL: "Induction of autologous graft-versus-host disease after autologous peripheral blood stem cell transplantation" JOURNAL OF ALLERGY AND CLINICAL IMMUNOLOGY, MOSBY - YEARLY BOOK, INC, US, vol. 103, no. 5, 1 May 1999 (1999-05-01), pages S457-S461, XP005178380 ISSN: 0091-6749

## Description

### Field of invention

The invention relates to methods for producing microvesicles, particularly microvesicles that are immunologically-matched autologous microvesicles which can be used in therapeutic and cosmetic and other applications.

### Background of the invention

### Microvesicles

Microvesicles (MVs) are membrane-bound packets of cytoplasmic material shed by cells in various physiological states, either natural or induced. Long regarded as cellular debris, recently these structures have generated an ever expanding literature supporting the hypothesis that microvesicles are an important inter cellular signalling mechanism (eg Ratajczak *et al.* 2006).

It has been reported that elevated levels of microvesicles are found in blood and other body fluids in various forms of pathology, most notably in cancers (reviewed Hoon & Taback, 2004). Consequently the value of microvesicles as a diagnostic tool is well established. More recently, it has been noted that microvesicles may be used to induce phenotypic changes in cells, specifically in stem cells (Aliotta *et al*., 2007; Ratajczak *et al*., 2006; Deregibus *et al*., 2007). Numerous compositions and methods for microvesicle production are available and known in the art. However, their origin (donor cell) is non-autologous with the intended recipient cell, thus their use in inducing phenotypic change may be problematic for various human and animal applications. Microvesicles will carry donor cell markers, and this may limit their use in clinical applications. Microvesicles are also known to transfer surface molecules target cells (see Derigibus *et al*. 2007 and references therein) which will lead to those target cells being tagged as "foreign" when non-autologous microvesicles are used. Further, it would be difficult to obtain significant numbers of autologous derived microvesicles to alter the phenotype of a recipient stem cell, or stimulate endogenous stem cell differentiation, migration and integration. Similarly, the use of microvesicles from a non-autologous source would carry significant risk of transferring pathogens from donor cell to recipient cells.

The present invention includes a method of stimulating stem cell differentiation using techniques known in the art. Harvesting microvesicles from such treated cells can then be applied to a further aliquot of the autologous stem cells. This method would enable large numbers of immunologically matched autologous microvesicles to be produced and applied to stem cells, progenitor cells or even fully differentiated cells *in vitro* and *in vivo*. While it is known that microvesicles can change cell phenotypes, the technical advance provided by the present invention is the ability to generate autologous matched microvesicles for communication with autologous cell sources. This improves efficacy and safety for the applications of microvesicles in regenerative medicine and many other applications.

### Disorders of cell deficiency and cell differentiation

A large number of human and animal diseases are caused by aberrant cell differentiation. The development processes that govern the ontogeny of a multicellular organism depend on the interplay between complex pathways of cell-to-cell signalling which gradually narrow the developmental potential of a cell from an original totipotent stem cell to a terminally differentiated mature cell which performs a specific function. However once a cell has successfully differentiated, in order for the health of the organism to be maintained, the differentiation of the cell needs to be maintained. One of the mechanisms by which this is achieved is that the cell is provided with an appropriate environment to maintain proper differentiation. Such an appropriate environment comprises appropriate signals from the extracellular matrix, other nearby cells (paracrine signalling), more distant cells (endocrine signalling) and from one cell to itself (autocrine signalling). The present invention is based on an appreciation that at least some of this signalling is conveyed from cell to cell by microvesicles.

The present invention is particularly applicable to the treatment of diseases and disorders caused by cell deficiency and diseases and disorders of cell differentiation.

Such diseases and disorders have in common the fact that they share an absence of appropriately differentiated cells and therefore proper cellular function. In diseases and disorders of cell deficiency, those cells are absent or depleted. In diseases and disorders of cell differentiation, those cells are present, but lack the correct differentiated phenotype.

### Summary of invention

The present invention provides a method for generating microvesicles autologous to stem cells. Such autologous microvesicles could then be used to induce changes in recipient cells with:
1. Minimal immunological consequence to a subsequent host of such cells after transplantation.
2. Reduce risk of pathogen transfer with microvesicles.
3. Enable autologous microvesicles and their derivatives to be used for a variety of clinical, veterinary, cosmetic, agricultural or research applications.

The principles of the present invention are summarised in the following diagram:-

**Diagram 1: Principles of the invention**

| **Stage 1** | |
|---|---|
| Microvesicles / Conditioned media / Cell extract, etc. known in the art to induce specific cell differentiation (Product A) harvested from a specific appropriate cell source eg cell line, primary tissue, foetal cell source, animal tissue, post mortem tissue, living donor etc. | |
| | ↓ |

| **Stage 2** | stem |
|---|---|
| Product (A) added to a first population of cells (B) to produce differentiated cells (C) these may be organotypic, system typical, or of a single cell phenotype for a specific pathology target, eg Oligodendrocytes, Neurones, Motor Neurones, Pancreatic islet cells, Type 1 or 2 Pneumocytes, Cardiomyocytes, Nephrons, etc. or a specific stem cell phenotype eg Embryonic stem cell, Foetal mesenchymal stem cell, Neural stem cell Mesenchymal stem cell etc. | |

| | |
|---|---|
| ↓ | |
| **Stage 3** | |
| Differentiated cells (C) maintained in appropriate culture and microvesicles (D) harvested. These microvesicles (D) would be autologous to the first population of cells (B). | |
| ↓ | |
| **Stage 4** | stem |
| Microvesicles D could then be used to alter the phenotype of further populations of cells, for example, stem cells, organs or tissues of the donor of stem cell B. Such cells would now have been modified by autologous microvesicles and could be used in a variety of applications where interventions using autologous materials would be safer or more desirable than foreign microvesicles. Or, Microvesicles (D) could be used to stimulate any form of natural or induced tissue repair, regeneration, rejuvenation by administration alone or in combination with other treatments such as cell therapy directly into the body avoiding the inherent immunological consequences of non-autologous microvesicles. | |

The final population of cells exposed to microvesicles (D) may then be used in a variety of applications including, but not exclusive to, regenerative medicine, surgery, cosmetics, veterinary medicine, reproductive medicine, agricultural and horticulture and clinical diagnostics.

According to certain embodiments of the disclosure these cells may be stem cells intended for therapeutic, non-therapeutic or cosmetic treatment, or banking of cells with a specific phenotype. Alternatively, these microvesicles (D) may be used as an injectable or implantable product to influence endogenous stem cells. Microvesicles (D) may also be stored or banked for subsequent use.

Microvesicles (D) may be used on their own or in combination with stem cells to treat disease, induce tissue/organ repair regeneration or rejuvenation. Alternatively, autologous microvesicles may be produced for cosmetic application.

According to a first aspect of the invention there is provided a method of producing a population of differentiated cells according to claim 1.

According to a second aspect of the invention there is provided a method of producing microvesicles according to claim 2.

According to a third aspect of the invention there is provided a method for generating a live cell product according to claim 7.

According the disclosure there is provided a method of treating a disease or disorder in a subject comprising:
a) inducing differentiation in a first population of cells by applying an inducer to said cells,
b) harvesting microvesicles produced from first population of cells, and
c) inducing differentiation in a second population of cells by applying said microvesicles or a derivative thereof to said second population of cells
d) administering said second population of cells or a derivative thereof to said subject,
wherein, said first population of cells and said second population of cells autologous to said subject and wherein the inducer is not present in said second population of cells or derivative thereof or is only present in trace amounts.

According the disclosure there are provided microvesicles and differentiated cells and derivatives thereof produced according to the first or second aspects of the invention.

According the disclosure there is provided a composition comprising microvesicles or differentiated cells of the invention or a derivative of either thereof in combination with a pharmaceutically acceptable carrier; and also a cosmetic agent comprising microvesicles or differentiated cells of the invention in combination with a cosmetic base.

According to the disclosure there is provided microvesicles, differentiated cells or a derivative of either thereof or a composition of the invention for use as a medicament.

According to the disclosure there is provided microvesicles, differentiated cells, or a derivative of either thereof or compositions of the invention for the manufacture of a medicament for the treatment of a disease or disorder of cell deficiency or a disease or disorder of cell differentiation.

According to the disclosure there is provided a method of providing non-therapeutic treatment to a subject comprising:
a) inducing differentiation in a first population of cells by applying an inducer to said cells,
b) harvesting microvesicles produced from said first population of cells, and
c) administering said microvesicles or a derivative thereof to a subject in need of said treatment,
wherein said first population of cells are autologous to said subject and wherein the inducer applied to said first population of cells in not present in said microvesicles or derivative thereof or is only present in trace amounts.

According to the disclosure there is provided a method of providing non-therapeutic treatment to a subject comprising:
a) inducing differentiation in a first population of cells by applying an inducer to said cells,
b) harvesting microvesicles produced from first population of cells, and
c) inducing differentiation in a second population of cells by applying said microvesicles or a derivative thereof to said second population of cells
d) administering said second population of cells or a derivative thereof to said subject,
wherein, said first population of cells and said second population of cells autologous to said subject and wherein the inducer is not present in said second population of cells or derivative thereof or is only present in trace amounts.

According to the disclosure there is provided a composition comprising stem cells in combination with microvesicles and a pharmaceutically acceptable carrier.

### Definitions

"Microvesicles" have at various times in their history been known as "exosomes", "secretory exosomes", "argosomes" and "microparticles". They can be distinguished from mere "cell debris", which is typically larger and denser. Microvesicles are lipid-bound entities produced by cells either spontaneously by living cells, at an accelerated rate by living cells subjected to certain stimuli or by artificial disintegration of cellular material. They are typically 10 nm to 1 pm in diameter (more typically about 30 to about 90 nm in diameter) with a buoyant density (typically 1 to 1.4 g/ml, more typically about 1.1 to about 1.2 g/m). They typically lack markers of lysosomes, mitochondia and cavedae.

"Autologous" refers to cells, tissues, proteins on microvesicles that are re-implanted into the same individual as they came from. In the context of the present application, the term is used to mean that the population of cells and/or microvesicles referred to as "autologous" to each other do not contain any material which could be regarded as allogenic or xenogenic, that is to say derived from a "foreign" cellular source.

A "cosmetic" method or product is not directed to the therapy of a disease but is, rather, directed to the improvement of an individual's aesthetic appearance, particularly the appearance of the skin or hair of an individual. Examples of cosmetic effects include a reduction in skin wrinkles, an increase in skin firmness, an increase in hair growth or shine, a reduction in grey hairs, a regrowth of hair in cases of baldness (especially male pattern baldness), an aesthetic enhancement of breast size or shape, and a reduction in cellulite. Cosmetic effects also include a reduction in hair growth (especially facial hair growth).

An "inducer" is any molecule or other substance capable of inducing a change in the fate of differentiation of a cell to which it is applied.

"Derivative thereof" includes a fraction or extract (especially those containing RNA and/or DNA and/or protein) of the original microvesicle or population of cells which retains at least some biological activity (especially the ability to induce differentiation and/or the ability to provide therapeutic benefit) of the original. Also included by the term are complexed, encapsulated or formulated microvesicles or cells (for example, microvesicles that have been encapsulated, complexed or formulated to facilitate uptake into recipient cells). According to certain embodiments, intact cells and microvesicles or derivatives containing intact cells or microvesicles are used. According to other embodiments derivatives such as lysates, lyophilates and homogenates may be used. Such derivatives have the advantage that they may be easier to store (for example they may be stored at room temperatures). The literature on live cell therapy demonstrates that cell homogenates and lysates can retain biological activity.

Derivatives of cells also include conditioned medium in which said cell has been grown.

In addition to cosmetic treatments, "non-therapeutic treatments" include treatments to animals to increase their commercial value or usefulness. For example, it includes treatment of food animals to promote growth and meat quality. Also included in the term is treatment to humans to enhance a specific physiological function, for example, a cognitive or physical property of the subject. For example, treatment to increase muscle mass may be desired in order to improve athletic ability; treatment to improve brain function may be desired to increase cognitive ability; and treatment to improve fertility may be desired to enhance reproductive function.

### Detailed description of the invention

The disclosure provides products, methods and medicaments for the production of autologous microvesicles for application to cell populations which may be banked, maintained in culture, transplanted to a recipient organism, or cells, tissues, organs or fluid environments which may be resident in a recipient organism. The methods provide techniques to generate autologous microvesicles to modify cells for various applications. The methods of the invention further reduce the risks associated with the use of non-autologous microvesicles. The microvesicles referred to in this patent application refer to any membrane bound particle, cytoplasmic or nuclear fraction, organelle, macromolecular, or molecular fraction or any other membrane bound or complexed entity extractable from media, cell homogenates, lysates, serum, body fluids, extra cellular fluids, cell cultures, organ cultures or embryo cultures or any cell, tissue, organ or organism. Further the term implies microvesicles of any size or weight. Still further, microvesicles in accordance with the invention may be further disrupted and or re-encapsulated in liposomes or complexed artificially with other molecular components to facilitate uptake into a recipient cell, tissue, organ, extracellular space, fluid or whole organism.

According to the present invention there is provided a method according to claim 1.

The first population of cells are a population of stem cells (for example, bone marrow derived stem cells). Preceding step a) of the above method of the first aspect of the invention, and optionally incorporated into that method there may optionally be the step of obtaining said first population of cells, for example stem cells from a subject. The first and second population of cells are autologous with respect to each other. The first and second populations of cells are obtained from the same individual subject. The first and second populations of cells may be obtained from the same subject at the same time or only a short interval apart. However, according to certain embodiments the first population of cells may be obtained from an individual several years (for example, more than 1,5, 10, 15 or 20 years) before the second population of cells is obtained. The first population of cells or microvesicles or a derivative thereof obtained from said population of cells having been banked in the intervening time.

The method of the first aspect of the invention provides the advantage that a population of cells (the so called "second population of cells") is induced to differentiate by contact with autologous material only (the microvesicles or a derivative thereof). Contact with the inducer in avoided. This means that the potential for the inducer to be immunogenic or an infection risk is eliminated or mitigated.

According to a second aspect of the invention there is provided a method according to claim 2.

Said method may be followed by a further step of:
c) lyophilising said microvesicles. Lyophilisation may be used to improve storage of said microvesicles.

The first population of cells are a population of stem cells (for example, bone marrow derived stem cells). Preceding step a) of the method of the first aspect of the invention, and optionally incorporated into that method there may optionally be the step of obtaining said first population of cells, for example stem cells from a subject in order to obtain said first population of cells.

The method of the second aspect of the invention provides the advantage that it provides a useful inducer of cell differentiation for application to a second population of cells which is autologous to the subject from which the first population of cells is obtained. The microvesicles produced and derivatives thereof are substantially free of the inducer used in step a). This means that the microvesicles and derivatives thereof lack any immunogenic material or infection risk which might be associated with the inducer.

According to the disclosure there is provided a method of treating a disorder or disorder in a subject comprising:
a) inducing differentiation in a first population of cells by applying an inducer to said cells,
b) harvesting microvesicles produced from said first population of cells, and
c) administering said microvesicles or a derivative thereof to a subject in need of said treatment,
said derivative may be a lysate, homogenate fraction, extract or lyophilate. wherein said first population of cells are autologous to said subject and wherein the inducer applied to said first population of cells in not present in said microvesicles or derivative thereof or is only present in trace amounts.

Preferably said subject is a subject of need of said treatment.

Preceding step a) of the method and optionally incorporated into that method there may optionally be the step of obtaining said first population of cells, for example stem cells from the subject.

Disorders of cell differentiation contemplated by the disclosure include and disease or disorder wherein cells of the body partially or fully loose their normal function required for health (as is the case with neurodegenerative disease) or wherein they acquire a function which is harmful to themselves, other cells or the organism (as is the case in cancer). Specific examples or diseases and disorders are given below.

According to the disclosure there is provided a method of treating a disease or disorder in a subject comprising:
a) inducing differentiation in a first population of cells by applying an inducer to said cells,
b) harvesting microvesicles produced from first population of cells, and
c) inducing differentiation in a second population of cells by applying said microvesicles or a derivative thereof to said second population of cells
d) administering said second population of cells or a derivative thereof to said subject,
wherein, said first population of cells and said second population of cells autologous to said subject and wherein the inducer is not present in said second population of cells or derivative thereof or is only present in trace amounts.

According to a third aspect there is provided a method according to claim 7. According to certain embodiments the derivative may be a conditioned medium, lyophilate, homogenate, fraction, extract or lysate.

Current methods of live cell therapy, which are based on the research of Dr Paul Niehans of Switzerland, involve the injection of animal tissue into humans for therapeutic purposes. The injections include lyophilates of animal tissue. The present invention provides an improvement to such methods because although animal tissue may be used as the initial inducer of differentiation of the first population of cells, the material to be introduced to the human subject is absent of animal material and therefore lacks the drawbacks associated with the use of animal material in humans.

Preferably said subject is a subject in need of said treatment.

Preceding step a) and optionally incorporated into that method there may optionally be the step of obtaining said first population of cells, for example stem cells from the subject.

Preferably said disease or disorder is a disease or disorder of cell deficiency or a disease or disorder of cell differentiation. Specific examples of such diseases are given below.

The disorder may be a traumatic injury or wound (for example an accidental or surgical wound) in need or repair and/or regeneration. Preferably said disease or disorder is a disease or disorder of cell deficiency, alternatively said disease or disorder is a disease or disorder of cell differentiation.

Specific diseases or disorders relating to the disclosure are given below along with suggested target tissue to be treated and suggested source material from which the inducer may be derived.

### Sources and Targets

The suggested source tissue for use in the present disclosure depends on the target tissue in which it is desired to effect a treatment response. The following lists describe example source tissues for specific target tissue and should be viewed as examples rather than being in any way limiting.

### i) Central Nervous System (CNS)

Tissues of the CNS will constitute the target tissue in various CNS-related conditions. Examples of CNS-related conditions that may be treated by the methods and products of the present invention include motor neurone disease, multiple sclerosis, degenerative diseases of the CNS, dementive illnesses such as Alzheimer's disease, age related dysfunction of the CNS, Parkinson's disease, cerebrovascular accidents, epilepsy, temporary ischaemic accidents, disorders of mood, psychotic illnesses, specific lobe dysfunction, pressure related injury, cognitive dysfunction or impairments, deafness, blindness anosmia, diseases of the special senses, motor deficits, sensory deficits, head injury and trauma to the CNS. Methods and products of the present invention may also be used to enhance brain function or ameliorate deficiencies at a functional level or to facilitate post surgical repair of the CNS.

Source tissue for the treatment of such CNS-related conditions includes whole CNS and subcomponents of the CNS such as whole brain, frontal lobes, parietal lobes, temporal lobes, limbic system, hippocampus, hypothalamus, thalamus, pituitary gland, pineal gland, sub-ventricular zone, olfactory bulb, any such defined anatomical region, nuclei or pathway (eg lateral geniculate nuclei, superior or inferior colliculi, dorsal root ganglia and substantia nigra), cerebellum, medulla oblongata, pons, ascending and/or descending tracts of the brain stem, whole or partial fractions of fore, mid, and hind brain structures, grey matter, white matter, specific cranial nerves, corpus callosum, optic chiasm and meningeal structures. In some embodiments, source tissue may comprise individual cell types or cultures enriched for specific cell types. Examples of such cell types include neurones, and specifically motor neurones, sensory neurones, inter-neurones, purkinje cells and pyramidal cells. Other examples include glia, and specifically astroglia, oligodendroglia, microglia, eppendymal cells, cells forming the blood brain barrier and the choroids plexi.

### ii) Cardiovascular system

Tissues of the cardiovascular system, and in particular the heart, may constitute the target tissue in various disorders of the cardiovascular system. Examples of disorders of the cardiovascular system that may be treated by the methods and products of the present invention include arrhythmias, myocardial infarction and other heart attacks, pericarditis, congestive heart diseases, valve-related pathologies, myocardial, endocardial and pericardial dysfunctions or degeneration, age-related cardiovascular disorders, dysfunctions, degeneration or diseases, sclerosis and thickening of valve flaps, fibrosis of cardiac muscle, decline in cardiac reserve, congenital defects of the heart or circulatory system, developmental defects of the heart or circulatory system, repair of hypoxic or necrotic damage, blood vessel damage and cardiovascular diseases or dysfunction (eg angina, dissected aorta, thrombotic damage, aneurysm, atherosclerosis, emboli damage and other problems associated with blood flow, pressure or impediment). Methods and products of the present invention may also be used to enhance cardiovascular function or health and to revascularise tissues. Moreover, methods and products of the present invention may be used to repair, modify, enhance or regenerate traumatic damage to the heart or blood vessels and as a technique to enhance the transplantation/implantation of a whole organ or its parts. Examples of this latter embodiment include heart transplantation, valve replacement surgeries, implantation of prosthetic devices and the development of novel surgical techniques.

Source tissue for the treatment of such disorders of the cardiovascular system includes whole heart or blood vessels, or subcomponents of the heart or blood vessels such as myocardium, endocardium, pericardium, cardiac muscle bundle, left and/or right atrial tissue, left and/or right ventrical tissue, valve derived tissue, trabeculae carneae, papillary muscles, chordiae tenineae, sinoatrial node, atrioventral node, atrioventricular bundle (bundle of His), aortic tissues, interventricular sulcus (including/excluding interventricular artery), superior or inferior vena cava and sympathetic and/or parasympathetic nervous tissues. Other suitable source tissue includes whole capillary, vein or artery tissue or sub-components of these tissues, including tissues of tunica interna (endothelium and/or subendothelial layer), internal elastic lamina, tunica media, external elastic lamina and tunica externa. In some embodiments, the source tissue may be continuous capillaries, fenestrated capillaries or sinusoids. In some embodiments, source tissue may comprise individual cell types or cultures enriched for specific cell types. Examples of such cell types include cardiac muscle cells, pukinje fibres and endothelial cells.

Conduction deficits may be treated with the use of tissue from the SA node or internodal pathways as source tissue. In another specific embodiment, focal calcification type arteriosclerosis may be treated with the use of smooth muscle cells of the tunica media as source tissue.

### iii) Respiratory system

Tissues of the respiratory system may constitute the target tissue in various disorders of this system. Examples of disorders of the respiratory system that may be treated by the methods and products of the present invention include damage, pathology, ageing and trauma of the nose and paranasal sinuses, nasopharynx, oropharynx, laryngopharynx, larynx, vocal ligaments, vocal cords, vestibular folds, glottis, epiglottis, trachea, mucocilliary mucosa, trachealis muscle, primary bronchi, lobar bronchi, segmental bronchi, terminal bronchioles, respiratory zone structures and plural membranes. Examples of such damage include obstructive pulmonary diseases, restrictive disorders, emphysema, chronic bronchitis, pulmonary infections, asthma, tuberculosis, genetic disorders (eg cystic fibrosis), gas exchange problems, burns, barotraumas and disorders affecting blood supply to the respiratory system. Methods and medicaments of the present invention may also be used to repair, modify, enhance or regenerate the respiratory system following damage. Moreover, methods and products of the present invention may be used as a technique to enhance the transplantation/implantation of whole respiratory structures or organs or their parts.

Examples of source tissue for the treatment of such disorders of the respiratory system includes the entire respiratory system and its whole parts or sub-components, such as nasopharynx, oropharynx or laryngopharynx, larynx, vocal ligaments, vocal cords, vestibular folds, glottis, epiglottis, trachea, mucocilliary mucosa, hyaline cartilages, arytenoids cartilage, cuneiform cartilage, corniculate cartilage, respiratory mucosa, submucosa, adventis, primary bronchi, lobar bronchi, segmental bronchi, terminal bronchioles, respiratory zone structures and plural membranes. In some embodiments, source tissue may comprise individual cell types or cultures enriched for specific cell types. Examples of such cell types include mucosal epithelial cells, pseudostratified columnar, columnar, cuboidal epithelial cells, smooth muscle, type I alveolar cells, type II alveolar cells, alveolar macrophages, pulmonary capillary, diaphragm tissues and intercostals muscle.

In a specific embodiment, cystic fibrosis may be treated with the use of tracheal lining mucosa as source tissue. Such a treatment may ameliorate the respiratory problems and damage associated with this condition. The treatment could be supplemented by the use of GI tract mucosa, thymus, thyroid and/or epithelial tissue of the reproductive tract as source of inducer in the methods to target specific deficits in function associated with the condition.

### iv) Gastrointestinal tract and associated glands

Tissues of the gastrointestinal tract (and associated glands) may constitute the target tissue in various disorders of the gastrointestinal tract. Examples of disorders of the gastrointestinal tract and associated glands that may be treated by the methods and medicaments of the present invention include disorders, damage and age related changes of both the gastrointestinal tract and the large accessory glands (liver and pancreas), salivary glands, mouth, teeth, oesophagus, stomach, duodenum, jejunum, ileum, ascending colon, transverse colon, descending colon, sigmoid colon, rectum and anal canal and enteric nervous system of the canal. In specific embodiments, these disorders, damage and age related changes include dental caries, periodontal disease, deglutition problems, ulcers, enzymatic disturbances/deficiencies, motility problems, paralysis, dysfunction of absorption or absorptive surfaces, diverticulosis, inflammatory bowel problems, hepatitis, cirrhosis and portal hypertension. Methods and medicaments of the present invention may also be used to repair, modify, enhance or regenerate the gastrointestinal tract following damage, or be used as a technique to enhance any of these processes following surgery, such as resection of the stomach, ileostomy and reconstructive surgery (eg ileoanal juncture). Examples of this latter embodiment include reconstructive surgery involving specific anatomical structures of the mouth, such as labia, vestibule, oral cavity proper, red margin, labial frenulum, hard palate palatine bones, soft palate, uvula, tongue, intrinsic muscles of the tongue and extrinsic muscles of the tongue.

Source tissue for the treatment of such disorders of the gastrointestinal tract and associated glands may include the entire gastrointestinal tract and its whole parts or sub-components, including the large accessory glands (liver and pancreas), salivary glands, mouth, oesophagus, stomach, duodenum, jejunum, ileum, ascending colon, transverse colon, descending colon, sigmoid colon, rectum and anal canal and enteric nervous system. Examples of other suitable source tissue include specific tissues or cells defined by structure or function such as mucosal tissue and glands, submucosa, submucosal glands, myenteric nerve plexus, submucosal nerve plexus, musclularis, serosa and specific glands of the digestive tract. In some specific embodiments, the source tissue may be obtained from specific oral structures to include labia, vestibule, oral cavity proper, red margin, labila frenulum, hard palate palatine bones, soft palate, uvula, tongue, intrinsic muscles of the tongue, extrinsic muscles of the tongue. In certain embodiments, source tissue may comprise individual cell types or cultures enriched for specific cell types.

Diverticulosis may be treated in methods employing tissue obtained from the whole colon or tissue extracted from muscularis and mucosa, or derivatives thereof, as the inducer.

### iv) Integumentary system

Tissues of the integumentary system may constitute the target tissue in various disorders of the integumentary system. Examples of disorders of the integumentary system that may be treated by the methods and medicaments of the present invention include disorders, damage and age related changes of the skin and integumentary system, such as age related decline in thickness or function, disorders of sweat gland and sebaceous glands, piloerectile dysfunction, follicular problems, hair loss, epidermal disease, diseases of the dermis or hypodermis, burns, ulcers, sores and infections. Methods and products of the present invention may also be used to enhance, regenerate or repair skin structures or functions, for example in plastic reconstruction, cosmetic repair, tattoo removal, wound healing, modulation of wrinkles and in the treatment of striae, seborrhoea, rosacea, port wine stains, skin colour and the improvement of blood supply to the skin. Moreover, methods and products of the present disclosure may be used to enhance skin grafts, surgical reconstruction, cosmetic surgical procedures, wound healing and cosmetic appearance.

Source tissue for the treatment of such disorders of the integumentary system includes whole skin, cultured skin, components of the integumentary system, the epidermis, dermis, hypodermis, subcutaneous fats, hair follicles, glands and associated structures. In some embodiments, source tissue may comprise individual cell types or cultures enriched for specific cell types.

Full thickness burns may be treated in methods employing the use of whole skin or derivatives thereof as the inducer.

### v) Musculoskeletal system

Tissues of the musculoskeletal system may constitute the target tissue in various disorders of the musculoskeletal system. Examples of disorders of the musculoskeletal system that may be treated by the methods and products of the present invention include disease, damage and age related changes of the musculoskeletal system. In some embodiment, these may be in components of the axial skeleton, including the skull, cranium, face, skull associated bones, auditory ossicles, hyoid bone, sternum, ribs, vertebrae, sacrum and coccyx. In other embodiments they may be in components of the appendicular skeleton, including the clavicle, scapula, humerus, radius, ulna, carpal bones, metacarpal bones, phalanges (proximal, middle, distal), pelvic girdle, femur, patella, tibia, fibula, tarsal bones and metatarsal bones. Methods and products of the present invention may also be used to correct problems associated with ossification and osteogenesis, such as intramembranous ossification, endochondral ossification, bone remodelling and repair, osteoporosis, osteomalacia, rickets, pagets disease, rheumatism and arthritis. Moreover, methods and products of the present disclosure may be used to treat disease, damage and age related changes of the skeletal muscle, elastic cartilages, fibrocartilages, long bones, short bones, flat bones and irregular bones.

Examples of source tissue for the treatment of such disorders of the musculoskeletal system include whole musculoskeletal system and its sub-components, such as specific bones or muscle-derived tissues. In some embodiments, source tissue may comprise individual cell types or cultures enriched for specific cell types. Examples of such cell types include individual musculoskeletal cell types.

### vi) Other systems of the body

Disorders of other systems of the body may be treated by the methods and products of the present disclosure. For example, the present disclosure may be used to enhance function or treat disease, damage and age related changes in other systems of the body, including special senses, endocrine system, lymphatic system, urinary system, reproductive system and alterations in metabolism and energetics.

Source tissue for the treatment of such disorders may be obtained from whole organs, sub-components of organs, specific cell types and groups of cells, wherein said source tissue is related to the relevant target tissue.

### vii) Treatment of general age-related degeneration

Methods and products of the present disclosure may be used to treat, ameliorate, reduce or compensate for general age-related degeneration. Similarly, methods and products of the present invention can be used to retain youthful functions of the body. Moreover, methods and products of the present invention may be used to treat specific age related system dysfunction, such as cognitive impairment, hearing loss, loss of visual activity, endocrine imbalances, skeletal changes and loss of reproductive function.

Source tissue for such uses may be obtained from whole organs, groups of organs, sub-components of such organ(s) and groups of cell types, wherein said source tissue is related to the relevant target tissue, as discussed above.

Typically said inducer is derived from the source tissue by making an extract, fraction, derivative, conditioned medium, lysate, lyophilate or homogenate of the source tissue. Alternatively, the source tissue may be used as an inducer substantially intact. Preferably said disease or disorder is cancer, for example, neuroblastoma.

Said disease or disorder is selected from a list consisting of:
- traumatic injury, radiation damaging, brain injury and age-related degeneration.

According the disclosure the disease or disorder is infertility or subfertility (in either males or females).

According to the disclosure there are provided microvesicles and differentiated cells produced according to the first or second aspects of the invention and also derivatives of said microvesicles and differentiated cells.

According to the disclosure there is provided a composition comprising microvesicles or differentiated cells produced according to the invention or a derivative of either thereof in combination with a pharmaceutically acceptable carrier. Specific examples of compositions are given below.

Compositions of the disclosure may comprise a spray, an impregnated wound dressing, an impregnated surgical suture, a tissue glue, an ointment or cream or a composition for injection.

The present disclosure also provides a cosmetic agent comprising microvesicles or differentiated cells of the invention in combination with a cosmetic base. Preferably said cosmetic base is a cream, ointment, bath gel, shower gel, soap, shampoo, spray, injectable agent or dermatological filler.

According to the disclosure there is provided microvesicles, differentiated cells or a derivative of either thereof composition produced according to the invention for use as a medicament.

Preferably said medicament is for the treatment of a disease or disorder or category of disease or disorder as described further herein.

According to there is provided microvesicles, differentiated cells, or a derivative of either thereof or compositions produced according to the invention for the manufacture of a medicament for the treatment of a disease or disorder of cell deficiency or a disease or disorder of cell differentiation.

Preferably said disease or disorder is as described further herein.

According the disclosure there is provided a method of providing non-therapeutic treatment to a subject comprising:
a) inducing differentiation in a first population of cells by applying an inducer to said cells,
b) harvesting microvesicles produced from said first population of cells, and
c) administering said microvesicles or a derivative thereof to a subject in need of said treatment,
wherein said first population of cells are autologous to said subject and wherein the inducer applied to said first population of cells in not present in said microvesicles or derivative thereof or is only present in trace amounts.

Preferably said non-therapeutic treatment is a cosmetic treatment.

Preferably said cosmetic treatment is a treatment for improving the appearance of the skin or hair of said subject.

According to the disclosure there is provided a method of providing non-therapeutic treatment to a subject comprising:
a) inducing differentiation in a first population of cells by applying an inducer to said cells,
b) harvesting microvesicles produced from first population of cells, and
c) inducing differentiation in a second population of cells by applying said microvesicles or a derivative thereof to said second population of cells, and
d) administering said second population of cells or a derivative thereof to said subject,
wherein, said first population of cells and said second population of cells autologous to said subject and wherein the inducer is not present in said second population of cells or derivative thereof or is only present in trace amounts.

Preferably said non-therapeutic treatment is a cosmetic treatment.

According to methods of the disclosure, and optionally incorporated into said methods there may optionally be the step of obtaining said first population of cells, for example, stem cells from the subject.

According to the disclosure there is provided a composition comprising stem cells in combination with microvesicles and a pharmaceutically acceptable carrier. In such a combination product said stem cells are preferably autologous to said stem cells. Said microvesicles are preferably derived from differentiated cells (as evidenced by the presence of markers of cell differentiation) and as such display different differentiation markers to the stem cells. Said stem cells are preferably bone-marrow derived stem cells and are preferably human.

The following description is applicable to all aspects of the invention. Where features of the invention are disclosed above in the context of one aspect of the invention, it is to be understood that they may also relate to other aspects of the invention.

Preferably said first and second populations of cells are animal cells, more preferably, human cells.

Said inducer is not autologous to said first population of cells, for example inducer may be xenogenic to said first population of cells.

Said first population of cells comprises stem cells.

Preferably said stem cells are bone marrow derived stem cells.

According to certain embodiments of the methods of the invention there is between steps a) and b), steps b) and c) and/or steps c) and d) the further step(s) of:
- banking the product of the immediately preceding step following by retrieving said product from said bank
- said banking may involve freezing said cells or microvesicles or alternatively freeze-drying or lyophillization of said cells or microvesicles.

Alternatively a derivative of said cells or microvesicles (for example a fraction, extract, homogenate, conditioned medium, lysate or lyophilate) may be banked.

According to certain embodiments of methods of the invention production of microvesicles by said first population of cells may be enhanced by one of the following techniques:
- stressing said first population of cells
- heat shocking said first population of cells
- killing said first population of cells
- changing the temperature, CO₂ concentration, O₂ concentration, medium composition or saline levels to which said first population of cells is exposed.

According to certain embodiments of methods of the disclosure which involve a step of administration of a product to a subject, said administration comprises or consists of intravenous administration.

Alternatively, said administration comprises or consists of surgical implantation. Alternatively, said administration comprises or consists of topical administration.

**Inducers of differentiation of first population of cells** (illustrated as Product A in diagram 1) applied to a population of stem cells to induce specific differentiation of the first population of cells. The inducers used in accordance with the invention may be any inducers of cell differentiation known in the art.

In a preferred case these inducers may be microvesicles harvested from a specific cell type, developing cell type (eg immature cells), regenerating cells of a specific type, or specific phenotype of stem cell or progenitor cell or any cellular, tissue, organ or whole body source.

These microvesicles may be harvested from the media used to grow or maintain the microvesicle generating cells. The microvesicles may be generated naturally by the cells or the cells may be induced to shed microvesicles by techniques known in the art. According to certain embodiments the inducer may be a nucleic acid vector used to express a specific nucleic acid, for example a transcription factor in the cells. When such an inducer is used, it should be noted that according to methods of the disclosure the transfected cell is not directly applied to the subject in need of treatment. Accordingly, the risks inherent in making genomic modifications of organisms is avoided.

The inducer is non-autologous.

According to certain embodiments, the inducer may be a known and characterised inducer of cell differentiation. However, such an inducer may not always be available or desirable. According to certain embodiments the inducer may simply be a differentiated cell type showing the same differentiation state which it is desired to produce in the second population of cells or a product derived from that differentiated cell type (for example, microvesicles conditioned medium or a cell lysate, extract or homogenate). For example, if the second population of cells is desired to be pancreatic beta cells, the inducer may be an extract of pancreatic beta cells or conditioned medium produced by cultured pancreatic beta cells or a cellular extract or lysate derived from pancreatic beta cells or microvesicles produced by pancreatic beta cells. Such an approach means that it is possible to use the method of the present invention to treat any disease or disorder of cell deficiency or cell differentiation. The only technical requirement is that the identity of the absent cell type or the cell type showing aberrant differentiation is known and that there is available a source of correctly differentiated healthy cells corresponding to that cell type. The correctly differentiated healthy cells used as or to derive the inducer may be obtained from the subject in need of treatment, alternatively they may be obtained from another individual. Preferably the inducer is derived from the same species as the subject to be treated. However according to certain embodiments, the inducer may be derived from a different species (for example for reasons of ease of availability of material). Further examples of source tissue for the inducer are given above in the discussion of diseases and disorders to which the present invention may be applied. The methods of the invention avoids exposing inducer material to the second population of cells, thereby problems with immune reactions and transmissions of zoonoses which are usually present when xenographic material is used as eliminated and thereby make a far wider range of inducer material available for use and make it easy for a person skilled in the art to select an inducer for virtually any differentiation pathway.

The inducer may be microvesicles (which may be non-autologous microvesicles) alternatively, the inducer may be conditioned medium, cell homogenates, or cell lysates.

In a further embodiment, the first population of cells may be induced to differentiate by conditioned media. Specifically, cells of a desired phenotype may be cultured in media; the media may then be collected and applied to stem cells to induce specific differentiation of the first population of cells.

In yet a further embodiment, the first population of cells may be induced to differentiate by co-culturing the cells with an appropriate tissue type. Still further, they may be induced to differentiate by exposure to cell extracts of a specific cell type or macromolecular fractions of such extracts. Yet further, the cells may be induced to differentiate by culturing them in contact with secretory factors generated by a desired cell type. In this method the secretory factors may be isolated from cells or generated from/by living cells introduced into the stem cell culture environments for example, but not limited to, well inserts, raft systems, separating membrane structures or any technique known in the art.

The inducer may be obtained from primary tissue sources, organs, cell lines, immortalised cells, foetal tissues, adult tissues, regenerating tissues, any type of stem cells, progenitor cells, and partially or fully differentiated cells or any other cell, organ, system or whole body source or placenta, umbilical cord or amniotic fluid. It is hypothesised that inducers derived from differentiated cells may be particularly suitable for use in methods suitable for the restoration of function of a similar specific differentiated cell type and that inducers derived from less differentiated cells (for example stem cells or foetal cells) may be particularly suitable for use in methods suitable for the restoration of function to a wider variety of cell types, for example for use in methods in reversing general age-induced damage.

In certain preferred embodiments the source of the inducer is human. In other embodiments the inducer may be from a non-human source. In other embodiments the inducer may be obtained from plants or other organisms. In preferred embodiments the inducer is from living tissue. In other embodiments the inducer may be obtained from dead tissues. In still further embodiments, damaged or regenerating tissues may be used.

### First population of cells

The first population of cells used in the methods of disclosure may be a population of stem cells or progenitor cells. The cells may be human, plant or non-human animal derived dependant upon the desired application. In a preferred embodiment the stem cells are stem cells derived from an adult, juvenile, infant, neonate or foetus. In other embodiments, the stem cells are derived from the umbilical cord, umbilical cord blood, amniotic fluid or placenta. In certain specific embodiments the stem cells may be adult bone marrow or blood stem cells collected from bone marrow aspirates or such cells mobilised into peripheral blood for collection by phlebotomy techniques known in the art, eg by use of GCSF etc. In other embodiments the stem cells are embryonic stem cells. Cell surface markers may be used to isolate and purify any stem cells used in the invention and the stem cells may be expanded in number prior to application of the inducer, or expanded subsequent to the application of the inducer or expanded while in the process of the addition of the inducer. The exposure to the inducer may be a single, multiple or continuous process over time.

The first population of cells are cultured *in vitro* using known techniques and exposed to the inducer *in vitro*. They are typically then cultured for a sufficient period of time for differentiation to take place or be induced (for example, 20 minutes to 1 hour, 30 minutes to 2 hours, 1 to 2 hours, 1 to 3 hours, 2 to 6 hours, 2 to 10 hours, 3 to 12 hours, 6 to 10 hours, 10 to 20 hours, 15 to 24 hours, 1 to 3 days, 2 to 5 days, 3 to 7 days, 5 to 14 days, 7 to 21 days, 14 to 21 days, 5 to 28 days, 21 to 28 days, 21 to 48 days or more than 14, 18, 21, or 28 days). Whilst full differentiation may take a relatively long time, the signal required to induce that differentiation may only need to be applied for a relatively shorter period of time.

### Ethical and legal constraints on source of cells

The inventor and applicant are fully aware of potential ethical and legal issues surrounding the use of stems cells and surrounding the use of material derived from embryos and foetuses. Those issues are particularly acute when the material is derived from animals, especially from humans. The inventor and applicant will comply with the recognised legal frameworks in each country in which they work. The applicant disclaims any subject matter excluded from patentability by statute on moral grounds or in the interests of public policy (*ordre public*). In particular, the applicant reserve the right to restrict any of the claims of this application (or parts of claims, or products recited in the claims) to non-animal or non-human applications, non-animal or non-human products or to the use of non-animal or non-human cells or cell-derived products, to disclaim the use of embryonic stem (ES) cells and to disclaim commercial and/or industrial uses of human embryos. Similarly, the applicant reserves the right to disclaim method of medical treatment carried out on the human or animal body in jurisdictions wherein such claims are restricted by law, by attaching a disclaimer to any claim or by specify that any step or combination of steps of any method disclosed herein are carried out for either a non-therapeutic purpose and/or are carried out *in vitro* rather than on the human or animal body (for example carried out on a sample or product previously obtained from a human or animal body or carried out *in vitro* prior to application to the human or animal body).

The stem cells of the present invention are stem cells which have not been derived from a human embryo.

### Obtaining Microvesicles

The differentiated first population of cells (C) obtained in steps of methods of the invention that correspond to stage 2 of diagram 1 is maintained in any appropriate culture conditions and microvesicles produced from them (D) harvested. These microvesicles (D) will be autologous to the first population of cells. Microvesicles according to the invention may be any membrane bound particles obtainable from the supporting media of the cells, or by artificially disrupting, damaging or stressing the cells in culture.

Microvesicles may be isolated from the culture in which they are produced by a variety of techniques known in the art (for example, differential centrifugation and/or density-gradient centrifugation). One such technique takes the media derived from the cell culture and centrifuges it twice at 1800 rpm for 20 minutes at 4 °C to remove cellular debris. The cell-free fluid or supernatant is then ultracentrifuged at 25,000 rpm at 4 °C for 3 hours. From this spin the supernatant is discarded, and the microvesicle containing pellet retained and resuspended in sterile Phosphate buffered saline. After treatment with or without 2% Triton X-100 at 4 °C for 30 minutes, the microvesicle suspension is centrifuged at 14,000 rpm at 4 °C for 1 hour. The microvesicles may then be stored at an appropriate temperature until use. In certain embodiments, the microvesicles or derivatives thereof may be stored long term by any freeze dry techniques eg lyophilisation under vacuum. The microvesicles generated and harvested in this stage are autologous to the first population of cells. Microvesicles may be harvested continually through the tissue culture process or at any specific time of the culture. Similarly, the cells generating the microvesicles may be passaged or frozen at any stage for subsequent use in generating autologous microvesicles.

The first population of cells, in common with all cells, will spontaneously be producing microvesicles into their culture medium. The rate at which microvesicles are produced may optionally be enhanced by special treatment of the cells, for example by exposure of the cells to a hypoxic or partially hypoxic environment.

### Cell and Microvesicles

Microvesicles are used to alter the phenotype a second population of stem cells. This second population of cells may be cells cultured *in vitro* or cells *in situ* in a subject to be treated. Such cells which have been modified by autologous microvesicles or derivatives thereof may be used in a variety of applications where interventions using autologous materials is safer or more desirable than foreign or non-autologous microvesicles. The microvesicles of the invention communicate, interact or deliver specific cellular information, instructions or products to the second population of cells, which may be stem cells, or differentiated cell, progenitor cells, egg cells, sperm cells or any other cell type or types *in vitro* or *in vivo,* such as to change the phenotype of the second population of cells, change the developmental stage of the second population of cells rejuvenate the second population of cells or otherwise modify the second population of cells. The microvesicles or derivatives thereof delivered to the second population of cells *in vitro* or *in vivo* may be enhanced by a composition or by preparing the microvesicles in appropriate gels, solutions, formulations, matrices, stents etc to facilitate communication with recipient cells.

According to certain embodiments, the autologous microvesicle enhanced or altered second population of cells may be used for any application. In certain preferred embodiments the cells may be stored for subsequent applications. The cells may be used whole or disrupted to produce a derivative thereof, for example, a cell extract, conditioned medium, lyophilate, homogenate or macromolecular fraction eg protein, peptides, RNA, DNA, lipids or combinations thereof for subsequent applications. Preparation of extracts or macromolecular fractions may be by any technique known in the art.

According to certain embodiments of the disclosure said second population of cells is *in situ* in a human or animal body. According to certain other embodiments the second population of cells is *in vitro* and the method of the disclosure optimally comprises the additional step of administering the second population of cells to a human or animal body following application of said microvesicles or derivative thereof to said second population of cells.

The microvesicles of the invention and/or the second population of cells and/or derivatives of either thereof may be used to stimulate tissue repair, regeneration, rejuvenation or enhancement by direct delivery to aged, damaged or compromised tissues either alone as autologous microvesicles or in combination with any other technique known in the art to facilitate tissue repair. Examples may include but are not limited to cell therapy, stem cell therapy, organ transplant, tissue transplantation, hormone therapy, surgical repair, cosmetic applications etc.

### Medicaments and methods for treating patients

The microvesicles, or second population of cells or derivatives of either thereof according to the disclosure may be used in the manufacture of medicaments and protocols to treat a number of disorders, where the origin of both the microvesicles and the second population of cells is preferably autologous to the subject of the treatment. The microvesicles, second population of cells or derivatives of either thereof may be delivered to or by any appropriate route of delivery or by surgical implantation. In certain preferred embodiments the recipient is the autologous human, animal or plant of the first population of cells was derived. In certain preferred embodiments the second population of cells, microvesicles or derivatives of either thereof are co-delivered, delivered subsequent, or delivered prior to other agents known to influence tissue regeneration, repair, rejuvenation or tissue enhancement (eg stem cells or any cell therapy).

The disclosure may employ a number of approaches to provide medicaments to treat disorders. In particular, administration of the products of the invention to a subject to be treated may be used for the following purposes:
- Administration of autologous cells treated or enhanced or altered by autologous microvesicles
- Use of microvesicles to transfer autologous surface marker molecules to non-autologous cells or tissue (for example, cells or tissue for use in xenographs or allographs) in order to make the non-autologous cells or tissues less likely to be rejected by the immune system. Such a treatment of non-autologous cells and tissues is likely to be temporary, but will give the transplant time to become established and for other mechanisms of immune tolerance to take place.
- Co-administration of autologous stem cells and autologous derived microvesicles
- Subsequent administration of autologous microvesicles after stem cell therapy either autologous or non-autologous
- Manufacture of autologous cell types from stem cells by the process of the invention for administration to subjects. These may be combination of cell types eg neurones, astrocytes, oligodendrocytes etc or single cell phenotype eg islet cells, oligodendrocytes, cardiomyocytes etc
- Delivery of natural or synthetic molecules of therapeutic importance *in vitro* and *in vivo* via autologous microvesicles manufactured in stage 3 (as illustrated in diagram 1) or stem cells altered by the manufactured microvesicles
- Manufacture of autologous whole cells, cell extracts, cytoplasm, cytoplasmic components or molecular fractions thereof for therapeutic, or any commercial application
- A method of introducing to cells, by autologous generated microvesicles, genes, DNA, RNA, proteins or peptides or any other molecule or combinations of molecules both *in vitro* and *in vivo*
- Any other application or potential application of microvesicle mediated inter cellular communication, delivery or cellular alteration where the use of autologous manufactured microvesicles may offer an advantage or be safer than non-autologous methods known in the art
- Administration of any integers of the invention to improve tissue repair, restoration, rejuvenation, regeneration or enhancement

Combinations of the above integers may also be employed.

In some embodiments it may be desired to use the invention to generate autologous microvesicles to induce the second population of cells (which are stem cells) to differentiate to a specific type of cell to ameliorate disease, repair damage or rejuvenate or regenerate tissues, organs or whole systems of the body. To achieve this, the invention may be used in a variety of ways, these include but are not limited to autologous stem cell therapy, stem cell banking, microvesicle banking and storage etc. In further detail of these embodiments, a patient may elect to have stem cells harvested from their body, or umbilical cord of a newborn infant or any other suitable donor source or cell line. Such cells would be expanded in the laboratory and divided into aliquots. One aliquot would be induced to differentiate to a desired cell type by methods known in the art. These cells would then be used to harvest microvesicles. Said microvesicles would now have membranes autologous to the donor of the stem cells. These microvesicles would then be applied to a second aliquot of stem cells from the same patient to induce the specific differentiation (autologous microvesicles on autologous cells). The differentiated stem cell could then be used directly in wholly autologous therapy or the cells or derivative banked in an appropriate cell bank for further use. A patient may elect to bank specific cell types or derivatives thereof in the case of specific disease or of a family history of particular diseases or may elect to bank critical cell types eg neurones, cardiomyocytes, pancreatic islet cells, pneumocytes, nephrons, hepatocytes, keratinocytes, muscle cells etc, thus having the ability to bank all critical cell types in an autologous form for subsequent acute or degenerative medical need. This would offer many advantages over current stem cell banking practices.

In another example, the patient may elect to store their own autologous stem cells and a range of autologous microvesicles or derivatives thereof which had preferably been produced from differentiated cells for later differentiation of their stem cells. Such an approach has the advantage that only stem cells will need to be maintained under conditions to ensure continued viability. Storage of microvesicles or derivatives therefore is likely to be less technically demanding because viability will not need to be maintained.

The microvesicles and derivatives thereof of the invention also offer the opportunity for direct application to ameliorate disease by direct injection into the body as they will have autologous membrane markers. This may be done alone or in combination with stem cell injections thus exploiting the full capacities of microvesicles without provoking immune reaction or rejection in the host. This may be used to direct stem cells more efficiently in terms of their differentiation and/or migration to damaged tissues and facilitate engraftment into the appropriate organ. The present invention provides many advantages over the use of non-autologous microvesicles in such embodiments.

In another example, the autologous microvesicles or derivatives thereof are used to alter the phenotype of cancer stem cells. Thus, the autologous microvesicles or derivatives thereof may be used to differentiate tumourous material into normal healthy tissues. In such an application, the microvesicles or derivatives thereof may be injected by any route, delivered surgically or injected directly into the tumour. In this application the microvesicles or derivatives thereof may be used on their own, in combination with cell therapy or as an adjunct to other therapy, eg chemotherapy or radiotherapy, surgical ablation etc. When used in combination with other therapies for cancer (for example, conventional chemotherapy or radiotherapy) the microvesicles or derivatives may be used for their anti-tumour activity (in which case the inducer used in methods to generate the microvesicles preferably has a phenotype corresponding to the original non-cancerous phenotype previously exhibited by the tumour) or alternatively or additionally the microvesicles or derivatives (or indeed differentiated cells of the invention) may be used (optionally in combination with stem cells) to repair or prevent damage to bystander cells and thereby reduce the side effects of the chemotherapy or radiotherapy (in which case the inducer used in methods to generate the microvesicles, differentiated cells or derivatives of either thereof is preferably has a phenotype corresponding to healthy bystander cells or is or is derived from bystander cells taken from the subject to be treated before chemo or radiotherapy has been started.

In a further example, a patient may elect to rejuvenate aged stem cells back to a younger form or phenotype eg adult bone marrow mesenchymal stem cells back to foetal mesenchymal stem cells for therapy or banking, thus improving their own autologous stem cells for regenerative or rejuventative therapy now or in the future. All integers of the invention may be used in anti-ageing medicine and may be used to prolong human life or reverse age related damage.

In a yet further specific embodiment, the disclosure may be used to improve reproductive tissues by replacement with autologous stem cells mediated; autologous microvesicles, or a combination thereof. This would be of great value in repair, regeneration or replacement of ovarian tissues or testicular tissues in the treatment of infertility.

The integers of the invention may be used to enhance repair, regeneration, and rejuvenation in any tissues of the body caused by trauma, disease, ageing or other effects on the body.

In a specific embodiment, the invention may be used to generate autologous cell extracts, whole cells, freeze dried cell products, cryonically preserved cells and products or any other product for use in the general field of organotherapy, live cell therapy or glandular therapy; broadly defined but not limited to, the use of cells or cell fractions currently obtained from foetal organs of animals to treat disease or produce an anti-ageing effect in humans. A novel aspect of the invention would be the manufacture of autologous live cell extracts which would be safer to use and have better effects in the recipient organism.

### Cell and microvesicle banking

According to certain preferred embodiments of the methods of the invention, there is between steps a) and b) and/or steps b) and c) and/or steps c) and d), the further steps(s) of
- banking the product of the immediately preceding step followed by relocating said product from said bank.

Preferably, said banking involves freezing said cells or microvesicles or derivative of either thereof. Alternatively, said banking involves freeze drying or lyophillization of said cells or microvesicles or derivative of either thereof.

### Compositions

The present disclosure provides pharmaceutical compositions. Such compositions comprise a therapeutically effective amount of a Therapeutic, and a pharmaceutically acceptable carrier. In a specific embodiment, the term "pharmaceutically acceptable" means approved by a regulatory agency of government or listed in the US Pharmacopeia, the European or UK Pharmacopeia or other generally recognised pharmacopeia for use in animals, and more particularly in humans. The term "carrier" refers to a diluent, adjuvant, excipient, or vehicle with which the Therapeutic is administered. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Examples of suitable pharmaceutical carriers are described in "Remington's Pharmaceutical Sciences" by E.W. Martin. Such compositions will contain a therapeutically effective amount of the Therapeutic, preferably in purified form, together with a suitable amount of carrier so as to provide the form for proper administration to the patient. The formulation should suit the mode of administration.

The composition may be formulated in accordance with routine procedures as a pharmaceutical composition adapted for intravenous administration to human beings. Typically, compositions for intravenous administration are solutions in sterile isotonic aqueous buffer. Where necessary, the composition may also include a solubilising agent and a local anaesthetic such as lignocaine to ease pain at the site of the injection.

The amount of the therapeutic of the disclosure which will be effective in the treatment of a particular disease or disorder will depend on the nature of the disease or disorder, and can be determined by standard clinical techniques. In addition, *in vitro* and *in vivo* assays may optionally be employed to help identify optimal dosage ranges. However, suitable dosage ranges for intravenous administration are generally about 20-500 micrograms of active component per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems. A typical dose for a human might be 10 pg to 10 mg, preferably 20 mg to 5 mg, preferably 40 pg to 2 mg, preferably 100 pg to 1 mg, preferably about 0.5 mg (calculated either per kg body weight or as total dose per individual).

Suppositories generally contain an active ingredient in the range of 0.5% to 10% by weight; oral formulations preferably contain 10% to 95% active ingredient.

Pharmaceutical preparations for topical and local use are, for example, for the treatment of the skin, lotions and creams which comprise a liquid or semi-solid oil-in-water or water-in-oil emulsion, and ointments (which preferably comprise a preservative). Suitable for the treatment of the eyes are eye drops which comprise the active ingredient in aqueous or oily solution, and eye ointments which are preferably manufactured in sterile form. Suitable for the treatment of the nose are aerosols and sprays (similar to those used in the treatment of the respiratory tract), coarse powders which are administered by rapid inhalation through the nostrils, and especially nose drops which comprise the active ingredient in aqueous or oily solution; suitable for local treatment of the buccal cavity are lozenges which comprise the active ingredient in a mass generally formed of sugar and gum arabic or tragacanth, to which flavourings may be added, and pastilles which comprise the active ingredient in an inert mass, for example of gelatine and glycerine or sugar and gum arabic.

Pharmaceutical preparations suitable for administration in the form of aerosols or sprays comprise, for example, suitable pharmaceutically acceptable solvent, such as, especially, ethanol and water, or a mixture of such solvents. They may, as necessary, comprise other pharmaceutical adjuncts, such as non-ionic or anionic surface-active agents, emulsifiers and stabilisers, and also active ingredients of other kinds, and especially advantageously they can be mixed with a propellant gas, such as an inert as under elevated pressure or especially with a readily volatile liquid, preferably a liquid that boils under normal atmospheric pressure below customary room temperature (for example from approximately -30 to + 10 °C), such as an at least partially fluorinated polyhalogenated lower alkane, or a mixture of such liquids. Such pharmaceutical preparations, which are used predominantly as intermediates or stock mixtures for the preparation of the corresponding medicaments in finished form, comprise the active ingredient customarily in a concentration of from approximately 0.1 to approximately 10% by weight, especially from approximately 0.3 to approximately 3% by weight. For the preparation of medicaments in finished form, such a pharmaceutical preparation is introduced into suitable containers, such as flacons and pressurised bottles, which are provided with a spray device or valve suitable for such purposes. The valve is preferably constructed in the form of a metering valve which on operation releases a predetermined amount of liquid, corresponding to a predetermined dose of the active ingredient. In the preparation of the finished medicament form, it is also possible for corresponding amounts of the pharmaceutical preparation in stock solution form and of the propellant to be introduced separately into the containers and to be mixed with one another only at that stage.

### Site of administration

Delivery of products to a subject to be treated may be achieved by providing the product locally, such as to the appropriate tissue or organ. For example, the administration of the product may be intravenous, rectal, oral, auricular, intraosseous, intra-arterial, intramuscular, subcutaneous, cutaneous, intradermal, intracranial, intratheccal, intraperitoneal, topical, intrapleural, intra-orbital, intra-cerebrospinal fluid, intranodal, intralesional, transdermal, intranasal (or other mucosal), pulmonary, or by inhalation to a site of interest. The product may, for example, be provided by local injection. The product may be provided by injection into a blood vessel or other vessel that leads to the desired target site. The product may be administered by local injection to the desired tissue. The product may be administered by any of the routes mentioned herein such as intra-muscular injection or by ballistic delivery. In some cases the localised delivery may be achieved because the product is provided in a form that specifically targets the product to the chosen cells. For example, the product may be provided in liposomes or other particles that have targeting molecules for the specific desired stem cell type. In many circumstances microvesicles and cells of the invention will naturally contain surface molecules for targeted delivery. In preferred embodiments the product may be administered via direct organ injection, vascular access, or via intra-muscular, intra-peritoneal, or sub-cutaneous routes.

### Veterinary use

Products and methods of the disclosure may specifically be applied in veterinary and agricultural applications to treat damaged tissues, ameliorate disease, reverse age related damage, improve reproductive function and extend reproductive life of valuable animals, for example endangered animals or stud animals.

### Cancer Treatment

The conventional view of tumourigenesis is that a cell must accumulate a number of harmful mutations to particular genetic loci (specifically tumour-suppressor and proto-oncogenes) and that such an accumulation of mutations is required and sufficient for the cell to become a tumour. Such a view underplays the role that the environment to which a cell is exposed to and the signals that a cell receives from other cells has in the development of cancer. It is more realistic to regard cells in a tissue as members of a cellular community wherein the each member is kept in line by appropriate signals from others cells in the community. It is hypothesised that many of those signals are conveyed by microvesicles. Example 6 demonstrates that cancer can be treated by providing microvesicles-conveyed signals to cancer cells to bring them back into line.

### Cosmetic uses

"Cosmetic use" includes products and methods involving placing active agents in contact with any part of the external surfaces of the subject's body (that is to say, the epidermis, hair system, nails, lips and external genital organs) or with the teeth and mucous membranes of the oral cavity with a view exclusively or mainly to cleaning them, perfuming them, changing their appearance, protecting them, rejuvenating them, keeping them in good condition or correcting body odours except where such cleaning, perfuming, protecting, changing, keeping or correcting is wholly for the purpose of treating or preventing disease.

Cosmetic uses and products include products and services that are intended as substitutes for invasive and semi-invasive aesthetic products and services (examples of such invasive and semi-invasive aesthetic products and services include (but are not limited to) wrinkle fillers, collagen fillers; body lifts; face lifts; eye-lifts; neck-lifts; arm lifts; hand lifts; and the like; as well as microdermabrasion; laser treatments; and, botox (or substitutes) injections; with a view to the user achieving some beneficial change in their human appearance.

### Examples

The invention is demonstrated by means of the following non-limiting examples:

### Example 1

### Improved differentiation of stem cells mediated by autologous microvesicles manufactured by a method of the invention compared to non-autologous microvesicles prepared by methods known in the art.

### Aim

To test the potential benefit of autologous microvesicles compared to non-autologous microvesicles in inducing stem cells to differentiate to a specific fate. It was hypothesised that the surface membrane properties of microvesicles may influence their efficiency in fusing with recipient cells. Further, that autologous microvesicles may show improved cell fusion and thus be a more powerful stimulant for specific stem cell differentiation.

### Stem cells

Stimulated bone marrow mesenchymal stem cells were harvested from peripheral blood of an adult male Hooded Lister rat following mobilisation of these cells using GCSF and plated into a 174 ml plastic culture flask (Falcon) in alpha-mem media with 1% penicillin and streptomycin and 10% foetal calf serum. Cells were incubated at 37 °C. After 48 hours, culture media was changed and cells washed with PBS. Adherent mesenchymal stem cells were retained and fed with fresh culture media. Cells were maintained in culture until they reached 75% confluence then passaged out to separate 6 well plates seeded for experimentation.

### Microvesicle preparation

In all groups microvesicles were isolated from cell conditioned media. Media was centrifuged twice at 1800 rpm for 20 minutes at 4 °C to remove cellular debris. The cell-free fluid or supernatant was then ultracentrifuged at 25,000 rpm at 4 °C for 3 hours. From this spin the supernatant was discarded, and the microvesicle containing pellet retained and resuspended in sterile phosphate buffered saline (PBS). After treatment with 2% Triton X-100 at 4 °C for 30 minutes, the microvesicle suspension was further centrifuged at 14,000 rpm at 4 °C for 1 hour. The product was stored at 4 °C until use.

### Experimental design

**Autologous microvesicle treatment group:** One six well plate of mesenchymal stem cells, prepared as above, was induced to differentiate to a neural phenotype by adding 1 ml of media conditioned by brain extract. Brain conditioned media was prepared by homogenizing a postnatal day 5 whole brain in 30 ml of a-mem medium for 15 hours. Supernatant was prepared by spinning the media for 20 min at 3000 rpm in a bench centrifuge. Clear supernatant was collected and was double filtered to ensure it was cell free. Conditioned media was left in the stem cell cultures for 24 hours, cells were then washed and placed in 3 ml of fresh media. After a further 24 hours the fresh media from each well of the neural induced stem cells was harvested for microvesicle extraction as above. These microvesicles would have the membrane profile of the original stem cells and thus be autologous in nature to the receiving stem cells.

**Nou-autologous microvesicle treatment group:** Microvesicles were prepared as in the above group but from stem cells derived from an unrelated Hooded Lister rat whose stem cells had been treated with an identical conditioned media to induce differentiation. These microvesicles were applied to another 6 well plate of the original stem cells and thus the microvesicles would be non-autologous to the final recipient cells.

### Non differentiated autologous stem cell generated microvesicles:

Microvesicles were prepared from undifferentiated autologous stem cells from media harvested as above and applied to a second autologous culture of stem cells.

All receiving stem cells were from the same original sample. 72 hours after treatment cells were subjected to immunohistochemical analysis using markers of neural differentiation (NeuN, Map2). The number of cells expressing markers was estimated visually by counting the number of expressing cells per visual field as a % of cells in that field.

### Results

| **% Neutral expressing cells** | **NeuN** | **Map2** |
|---|---|---|
| **Autologous Microvesicle Group** | **79%** | **81%** |
| **Non-autologous Microvesicle Group** | **26%** | **22%** |
| **Stem cell Microvesicle Group** | **0%** | **0%** |

**Discussion:** It can be clearly seen that the use of microvesicles can induce differentiation of stem cells confirming the findings of (Aliotta *et al.* 2007; Ratajczak *et al*., 2006). However, the use of microvesicles generated by a method of the invention, a two stage process of inducing stem cells to differentiate to a specific cell type, then harvesting microvesicles from these cells after differentiation for induction of a second aliquot of autologous stem cells, produces a profoundly better inductive signal. Further, such treated stem cells would be autologous to a specific organism, and have been induced by autologous microvesicles thus providing a stem cell and/or stem cell generated product which would have any advantages in many varied applications both *in vitro* and *in vivo.* It was also noted that undifferentiated stem cell generated microvesicles had a significant proliferative effect on stem cells in culture.

### Example 2

### Autologous microvesicle primed stem cell induced repair in aged rat compared to existing non-autologous equivalent methods.

Having established an advantage to the methods claimed in the invention for autologous microvesicle induced differentiation, their efficacy in an *in vivo* model was explored. It was hypothesised that non-autologous microvesicle induced differentiation, known in the art may change the surface characteristics of a recipient cell by the nature of their contributed cell membrane. Thus, such stem cells may not be capable of reaching their full potential of repair, regeneration or other reparative functions as many cells may be destroyed by the host immune system.

The test employed was a cognitive test of spatial memory, the Morris water maze. Aged rats are known to be poor at both learning and recalling this task. In brief the water maze is a plastic tank 1.8 m in diameter filled with 21 °C water. The water is made opaque by the addition of powdered milk to conceal a hidden submerged escape platform located in a fixed position in the pool relative to obvious landmarks in the laboratory. Each rat in training received 3 trials of maximum 60 second duration per day for 3 consecutive days. On each trial the latency to locate the platform is recorded. If the animal fails to locate the platform it is gently guided to the platform and is allowed 15 seconds on the platform to orient itself. Animals successfully locating the platform are also allowed the same orientation period. At the termination of each trial, animals are returned to a communal drying cage for a rest period of 15 minutes prior to their next trial. At the end of the three day training period animals were maintained in communal home cages before having a single recall test 1 month post training to assess long term memory of the task. Young rats readily learn this task over the 9 acquisition trials, and show excellent recall in long term testing. Old rats show poor acquisition evidenced by lack of improvement over trials in latency to find the hidden platform and poor long term recall because of age related neurological degeneration.

Animals used in this study were all Sprague Dawley rats aged between 501 and 546 days.

### General methods:

Three groups of animals were studied:
Group 1: Autologous microvesicle stimulated autologous stem cells
Group 2: Non-autologous microvesicle stimulated autologous stem cells
Group 3: Autologous stem cells only

Stem cells were injected intravenously into the tail vein of the autologous animal. 300,000 approx. cells in a 0.3 ml injection using a 27G needle.

### General procedure:

Autologous mesenchymal stem cells were obtained from peripheral blood of each identified animal. Cells were maintained in culture and expanded into 175 ml plastic culture flask as outlined in example 1. For each animal in each group the method was as follows:
**Group 1:** One flask of autologous stem cells (stem cells A) was primed to differentiate into neural cell phenotypes by the administration of adult brain conditioned media as described above. After 72 hours media was collected from these cells and processed for microvesicles as described. Microvesicles from induced stem cell population A were resuspended in fresh media and applied to a second autologous stem cell population (stem cells B). These microvesicles should contain the inductive neural differentiation signal as they are now generated from neural phenotype but their membranes will be autologous to the second batch of stem cells (B) and the intended host.
**Group 2:** One flask of non-autologous stem cells (stem cells A) was primed to differentiate into neural cell phenotypes by the administration of adult brain conditioned media as described above. After 72 hours media was collected from these cells and processed for microvesicles as described. Microvesicles from induced stem cell population A were resuspended in fresh media and applied to a second (host autologous) stem cell population (stem cells B) identical to group 1. These microvesicles should contain the inductive neural differentiation signal as they are now generated from neural phenotype but their membranes will be partly non-autologous to the second batch of stem cells (B) and the intended host.
**Group 3:** Autologous stem cells (stem cells B) were cultured as described but not treated with any microvesicles.

All three groups were compared to non treated age matched control animals. Each group comprised eight identified animals.

Testing of the animals commenced 24 days post injection.

### Results and discussion

Untreated aged rats showed no significant improvement in locating the platform over the 9 acquisition trials, with a mean latency on trial 9 of 56 seconds. Similarly, they showed no long term memory at 1 month (mean 60 seconds). Interestingly, autologous stem cell treated animals (group 3) did show some improvement over training (mean at trial 9 was 34 seconds) but showed poor recall over 3 days and this learning curve was poor compared to historical data for young rats trial 9 mean approx. 6-8 seconds). Again, group 3 showed no long term recall at the 1 month retest.

Animals in group 2, treated with autologous stem cells primed with non-autologous microvesicles did show some improvement by trial 9 (mean 24 seconds) but showed poor recall at the 1 month retest (52 seconds). Animals in Group 1, treated with autologous stem cells primed with autologous microvesicles showed excellent acquisition of the task over 9 trials (trial 9 mean 4 seconds). Further, they showed excellent recall of the task at the 1 month retest (mean 11 seconds).

These data demonstrate convincingly the benefit of controlling stem cells by the novel approach of priming differentiation using autologous microvesicles. Their efficacy in this pre clinical model of dementia was significantly improved (p<0.0001) compared to existing microvesicle mediated stem cell technology. Similarly, results have been obtained in *in vivo* pancreatic repair models, where autologous microvesicle treated autologous stem cells produced by the method of the invention produced normal blood sugar levels in rats which were maintained indefinitely (over 4 months). Animals treated with non-autologous microvesicle stimulated stem cells showed some normalisation of blood sugar for two weeks post injection but then the stem cell mediated effect failed.

It is apparent from examples 1 and 2 that the autologous nature of the microvesicles to the second batch of stem cells (and the eventual host animal) improves communication of signal (eg fusion), enhances the microvesicle induced change of cell characteristic, and enhances engraftment of the stem cell in the host thus yielding a better reparative effect on tissues *in vivo.*

### Example 3

Recently, much interest has focused on microvesicles in a variety of areas of biological and medical science as a diagnostic, an inducer of stem cell differentiation and as mediators of various biological effects. All existent technology for cell modification relies on non-autologous microvesicles because autologous microvesicles would require some of the patients' damaged tissues to be removed and cultured *in vitro* to generate autologous microvesicles. This invention surmounts that issue with a novel two step approach. Further, microvesicle methods currently known in the art could not generate products which could be injected into a host organism as the microvesicles would mostly be destroyed by immune reaction. The invention disclosed in this patent application gets around that problem and provides methods to investigate microvesicle mediated direct repair in an organism. Thus a third example is provided investigating the potential of autologous microvesicles as direct mediators of tissue repair, regeneration or rejuvenation. This novel application of microvesicle technology using autologous stem cells primed to differentiate into specific cell types as generators of autologous membrane coated microvesicles for various applications in a host organism offers a host of applications in regenerative medicine.

To this end, example 2 was repeated with three groups of 8 aged (569-600 day old) rats treated as follows:
**Group 1:** Microvesicles were prepared from embryo rat (non-autologous) day 18 brain tissue maintained in tissue culture by standard culture techniques. Media was collected and microvesicles harvested as described above. These non-autologous microvesicles were resuspended in media and applied for 24 hours to a population of host autologous stem cells derived as described above. These microvesicle treated autologous stem cells were observed to differentiate to a neural like phenotype and their media was harvested 4 days after treatment. The media was collected, filtered and the cell free media used to generate microvesicles of a neural cell phenotype encapsulated in an autologous cell membrane. The resultant microvesicles were resuspended in 0.3 ml PBS and injected into tail veins of the appropriate autologous host animal.
**Group 2:** Microvesicles were prepared from embryo rat (non-autologous) day 18 brain tissue maintained in tissue culture by standard culture techniques. Media was collected and microvesicles harvested as described above. These non autologous microvesicles were resuspended in PBS and used for direct injection to a non autologous host animal in a 0.3 ml tail vein injection.
**Group 3:** Vehicle injection control animals. These animals received a tail vein injection of 0.3 ml PBS.

Training on the Morris water maze was as reported in the previous example and commenced 14 days post injection.

### Results and discussion

Group 3 animals showed no learning evidenced by any savings in latency to reach the hidden platform (Trial 9 mean 57 seconds). Similarly, they showed no long term memory of the training assessed 1 month post training (retest trial mean 60 seconds). Further, the non-autologous microvesicle treated group also showed no effect on training (Trial 9 mean 52 seconds) and no effect on long term memory (mean 60 seconds). However, excellent learning was found in the autologous microvesicle group (Trial 9 mean 5 seconds) with clear evidence of improved long term memory function (8 seconds at the 1 month retest).

These data clearly show that autologous microvesicles manufactured by the methods of the invention show an excellent ameliorative effect on age related cognitive decline. To my knowledge this is the first such demonstration of direct microvesicle mediated tissue repair *in vivo* and is made possible by the autologous membrane structure.

This experiment, along with others from our laboratory (unpublished), shows a promising new approach to various areas of regenerative and rejuvenative medicine as well as other applications such as, but not restricted to, cosmetics. It is concluded that the autologous nature of the generated microvesicles avoids detection by the immune system of the host, and the large numbers generated *in vitro* show the true potential of microvesicles. These data also complement the increasing interest in circulating nucleic acids in serum and plasma. Recent reviews (eg Hoon & Taback, 2004) show that microvesicles survive prolonged periods in the blood of animals and humans when released by damaged or cancerous tissues and offer many interesting hypotheses of why these microvesicles are released. My hypothesis is that such membrane bound particles are part of a natural repair process which can be significantly enhanced by this technology.

### Example 4

One area of historical interest to regenerative medicine is the now almost abandoned area of live cell therapy. This technique pioneered by Prof. Neihans in Switzerland in the 1930s and practised in many countries ever since, uses the principle of "like cures like". Such therapies involve injecting (intra muscular or subcutaneous) cellular homogenates (fresh or freeze dried) derived from embryonic animals (eg reviewed Schmid & Stein, 1967). Despite an expansive literature from European and Russian practitioners, this approach known as live cell or organotypic therapy continues to meet resistance by main stream medicine due to the xenographic nature of the injected material. The present invention, however, provides a novel method for generating various live cell products from microvesicle mediated cell transformation where the transformed cell could be homogenised, lysed or otherwise disrupted to produce autologous live cell products for various applications.

Example 4 investigated this particular application by manufacturing live cell therapy products from transformed autologous stem cells in an animal model of motor neurone disease.

The mouse model employed in this example was the SOD1 mouse strain which develops an aggressive form of motor neurone disease resulting in paralysis and early death usually by 140 days of age. A population of these animals was maintained in a breeding colony in our facility. Animals selected for inclusion in the study developed hind limb paralysis by 80 days of age. These animals were divided into three experimental conditions as follows:
**Group 1:** 20 animals had autologous bone marrow mesenchymal stem cells stimulated into peripheral blood, harvested and expanded as reported in previous examples. Cultured mesenchymal stem cells were maintained in 175 ml plastic culture flasks and treated with spinal cord tissue conditioned media derived from embryonic (day 16) foetuses for 24 hours. Conditioned media was filtered and centrifuged as described in example 1 to ensure it was cell free prior to application to autologous stem cells. The treated stem cells appeared to undergo morphological changes resembling neural tissues after 48 hours. At this stage media was harvested and microvesicles prepared as stated above. The microvesicle fraction (now with autologous membranes) was resuspended in media and applied to a second population of autologous stem cells for 48 hours. This second population of autologous stem cells showed extensive neural differentiation. Cells were removed from the culture environment by cell scraper and homogenised in sterile PBS for subsequent injection to the original donor of the stem cells.
**Group 2:** 20 animals were treated with traditional cell therapy product namely, homogenised embryonic (day 16) foetal spinal cord tissue if they met inclusion criteria.
**Group 3:** 20 animals meeting paralysis criteria by 80 days received a PBS only injection.

All injections were performed sub-cutaneously at day 80 with a total injected volume of 1 ml mediated in pinched skin of the neck. The number of animals surviving each day post injection was recorded and each animal was assessed for paralysis/improvement by a walk test every day post injection.

### Results & Discussion

Data summary following injection at day 80.

**Number of surviving animals (number showing improvement of paralysis)**

| Age | 80 | 100 | 120 | 150 | 180 | 200 | 250 | 300 days |
|---|---|---|---|---|---|---|---|---|
| Group 1 | 20(0) | 20(5) | 20(9) | 20(11) | 19(17) | 18(15) | 15(15) | 15(15) |
| Group 2 | 20(0) | 17(0) | 13(0) | 8(2) | 1(0) | 0(0) | 0(0) | 0(0) |
| Group 3 | 20(0) | 19(0) | 10(0) | 0(0) | 0(0) | 0(0) | 0(0) | 0(0) |

The SOD1 mouse is a well established animal model of aggressive motor neurone disease. Autologous live cell therapy products manufactured by the invention showed extensive amelioration of disease in group 1 compared to both traditional live cell treated (group 2) and vehicle control (group 3). These results show clearly, potential new approaches to the interesting results obtained with various live cell products in the past, and offer the advantage of reducing the risk of cross species infection, immunological crisis, etc.

In this application of the invention is the potential to produce fresh autologous live cell extracts, frozen, lyophilised or other stored live cell extracts. It offers the further potential of using the method to produce organotypic, multiple organotypic or individual cell type specific extracts for application as a product by practitioners in live cell therapy thus allowing this interesting area of medicine to realise its full potential with improved efficacy and greater safety.

### Example 5

The above experiment was repeated without the second phase of treating a second population of autologous stem cells ie using non-autologous or xenogenically sourced material to prime autologous stem cells to induce differentiation then homogenise the differentiated stem cells to provide a live cell therapy product. Animals selected for inclusion in the study developed hind limb paralysis by 80 days of age. These animals were divided into three experimental conditions as follows:
**Group 1:** 20 animals had autologous bone marrow mesenchymal stem cells stimulated into peripheral blood, harvested and expanded as reported in previous examples. Cultured mesenchymal stem cells were maintained in 175 ml plastic culture flasks and treated with spinal cord tissue conditioned media derived from embryonic (day 16) foetuses for 24 hours. Conditioned media was filtered and centrifuged as described in example 1 to ensure it was cell free prior to application to autologous stem cells. The treated stem cells appeared to undergo morphological changes resembling neural tissues after 48 hours. Cells were removed from the culture environment by cell scraper and homogenised in sterile PBS for subsequent injection to the original donor of the stem cells.
**Group 2:** 20 animals were treated with traditional cell therapy product namely, homogenised embryonic (day 16) foetal spinal cord tissue if they met inclusion criteria. **Group 3:** 20 animals meeting paralysis criteria by 80 days received a PBS only injection.

All injections were performed sub-cutaneously at day 80 with a total injected volume of 1 ml mediated in pinched skin of the neck. The number of animals surviving each day post injection was recorded and each animal was assessed for paralysis/improvement by a walk test every day post injection.

### Results & Discussion

**Number of surviving animals (number showing improvement of paralysis)**

| Age | 80 | 100 | 120 | 150 | 180 | 200 | 250 | 300 days |
|---|---|---|---|---|---|---|---|---|
| Group 1 | 20(0) | 20(5) | 20(6) | 20(19) | 19(12) | 18(12) | 15(11) | 13(11) |
| Group 2 | 20(0) | 12(0) | 9(0) | 8(2) | 2(0) | 0(0) | 0(0) | 0(0) |
| Group 3 | 20(0) | 19(0) | 11(0) | 0(0) | 0(0) | 0(0) | 0(0) | 0(0) |

Autologous stem cells can be induced to differentiate by conditioned media. These cells can then be homogenised and used for direct injection to homologous host to ameliorate damage. This method of the invention can clearly be seen to be advantageous to traditional methods again, with fewer risks than traditional methods known in the art.

### Example 6

Autologous microvesicle effects on cancer.

The methods of the invention provide interesting opportunities to develop products useful in the management of cancer. Microvesicles are known to be shed by cancer cells. Similarly, it is well established that microvesicles circulating in blood may be a useful diagnostic for cancer (Hoon & Taback, 2004). Further, it has been established that cancers of various types contain stem cells. Given these properties of both microvesicles and cancers, a programme of research was initiated in my laboratory to study if cancers could be differentiated back to normal tissues by the methods of the invention.

A commercially available tumour cell line B16 melanoma was acquired and cultured in 75 ml tissue culture flasks as per suppliers' instructions. This particular cell line of melanoma, actively secretes melanin into the media which turns a distinctive black colour. Further, on inoculation into SCID immuno-deficient mice they produce palpable and measurable tumour masses at the site of injection.

In the initial phase of the experiment, melanoma cells were passaged into 6 well plates after initial expansion. Seeding density was 200,000 cells in 3 ml of media per well. Two groups of cells were maintained in 6 well formats as follows:
**Group 1:** Melanoma cells were treated with microvesicles derived from stem cells induced to differentiate into muscle by harvesting microvesicles from a primary culture of muscle cells. Microvesicles were prepared as described in example 1 and applied to the tumour line in 1 ml of media for 24 hours.
**Group 2:** Melanoma cells were maintained as per manufacturer's instruction and were not treated.

Five days after treatment, cells were fixed and stained for immunohistochemistry for muscle specific marker proteins (myosin heavy chain antibody) and inspected visually for obvious signs of muscle differentiation.

### Results & Discussion

Group 2 cells maintained their tumour phenotype by visual inspection and discolouration of media. However, after 5 days media in Group 1 was not discoloured and clear evidence of muscle like differentiation of the melanoma cells was evidenced. Further, original melanoma cells in Group 1, in all six wells, strongly expressed myosin heavy chain marker. This suggests that the melanoma cells have differentiated into muscle cells using the methods of the invention.

To further test this, cells extractable from all treated wells were harvested by cell scraper and inoculated intramuscularly into the right thigh of two groups (n=3) of SCID mice.

Untreated tumour cells produced an obvious tumour in 6 days. No palpable mass was evidenced in group 1 treated melanoma cells at any stage of the study. These data suggest a novel approach to tumour, namely differentiating tumours back to a normal cell type rather than the traditional approach of trying to inefficiently kill the tumour cell, eg chemotherapy, radiotherapy. Thus it is illustrated that microvesicles manufactured from normal stem cells may have beneficial effects in the ontogeny of neoplasia.

These examples provided in the drawings sections show novel methods for the production of autologous microvesicles from stem cells for various applications, such microvesicles may be used directly *in vitro* or *in vivo,* or in further embodiments of the invention, may be used on a second population of autologous stem cells to produce products of value to various commercial applications in any field of medicine, veterinary practice, cosmetics, rejuvenation, reproductive technologies and other fields of commercial and scientific endeavour. These examples are provided to illustrate some aspects of the invention, but are not restrictive and the invention may be used in any field where it provides a novel solution to a problem.

### References

Aliotta, J.M. et al. (2007) Alteration of marrow gene expression, protein production and engraftment into lung by lung derived microvesicles: a novel mechanism for phenotype modulation. Stem cells 25(9):2245-56
Hoon, D.S.B. (2004) Circulating nucleic acids in plasma/serum III and serum proteomics. (Eds). Annals of the New York Academy of Sciences. 1022:1-8
Ratajczak, J. et al. (2006) Embryonic stem cell-derived microvesicles reprogram haematopoietic progenitors: evidence for horizontal transfer of mRNA and protein delivery. Leukaemia. 20, 847-856.
Schmid, F. & Stein, J. (1967). Cell research & cellular therapy. Ott Publishing House. Switzerland.
Deregibus, C. (2007). Endothelial progenitor cell-derived microvesicles activate an angiogenic program in endothelial cells by a horizontal transfer of mRNA. Blood: 110(7): 2440-2448

## Claims

1. An in vitro method of producing a population of differentiated cells comprising: a) obtaining a first population of stem cells which have not been obtained from a human embryo from a subject; b) inducing differentiation in said first population of cells by applying an inducer to said cells which is not autologous, c) harvesting microvesicles produced therefrom which are free of the inducer of step b); d) obtaining a second population of stem cells which have not been obtained from a human embryo from said subject which is autologous to said first population of cells; and e) inducing differentiation in said second population of cells by applying said microvesicles from step c) to said second population of cells, whereby the second population of cells is induced to differentiate by contact with autologous matched microvesicles and whereby contact with the inducer of step a) is avoided.

2. An in vitro method of producing microvesicles comprising: obtaining a first population of stem cells which have not been obtained from a human embryo from a subject; b) inducing differentiation in a first population of stem cells which have not been obtained from a human embryo by applying an inducer to said cells which is not autologous, and c) harvesting microvesicles produced therefrom which are substantially free of the inducer of step a), wherein said first population of cells is autologous to said microvesicles.

3. The method according to claim 1 or 2, wherein said first population of cells are human cells.

4. The method according to any preceding claim, wherein said first population of cells comprises mesenchymal stem cells.

5. The method according to any preceding claim, wherein said stem cells are bone marrow derived stem cells.

6. The method according to any preceding claim, wherein production of microvesicles by said first population of cells is enhanced by one of the following techniques:
• stressing said first population of cells
• heat shocking said first population of cells
• killing said first population of cells
• changing the temperature, CO₂ concentration, O₂ concentration, medium composition or saline levels to which said first population of cells is exposed.

7. A method for generating live cell product from microvesicle mediated cell transformation comprising: a) obtaining a first population of stem cells which have not been obtained from a human embryo from a subject; b) inducing differentiation in said first population of cells by applying an inducer to said cells which is not autologous, c) harvesting microvesicles produced therefrom which are free of the inducer of step b); d) obtaining a second population of stem cells which have not been obtained from a human embryo from said subject which is autologous to said first population of cells; e) inducing differentiation in said second population of cells by applying said microvesicles from step c) to said second population of cells; and f) disrupting the transformed cells of step e) to provide autologous live cell product, whereby the second population of cells is induced to differentiate by contact with autologous matched microvesicles and whereby contact with the inducer of step a) is avoided.

8. The method of claim 1, further comprising the step of banking the first population of cells after step a), or banking the microvesicles after step c).

9. The method of claim 2, further comprising the step of banking the first population of cells after step a), or banking the microvesicles after step c).

## Patentansprüche

1. In vitro Verfahren zur Erzeugung einer Population von differenzierten Zellen mit den folgenden Schritten: a) Gewinnen einer ersten Population von Stammzellen, die nicht von einem menschlichen Embryo gewonnen worden sind, von einem Versuchsobjekt; b) Induzieren einer Differenzierung in die erste Zellpopulation, indem ein Induktor auf die Zellen aufgebracht wird, der nicht autogen ist; c) Ernten von daraus erzeugten Mikrovesikeln, die frei von dem Induktor aus Schritt b) sind; d) Gewinnen einer zweiten Population von Stammzellen, die nicht von einem menschlichen Embryo gewonnen worden sind, von dem Versuchsobjekt, die gegenüber der ersten Zellpopulation autogen ist; und e) Induzieren einer Differenzierung in die zweite Zellpopulation, indem die Mikrovesikel aus Schritt c) auf die zweite Zellpopulation aufgebracht werden, wobei die zweite Zellpopulation zur Differenzierung durch den Kontakt mit entsprechenden autogenen Mikrovesikeln induziert wird und wobei ein Kontakt mit dem Induktor aus Schritt a) vermieden wird.

2. In vitro Verfahren zur Erzeugung von Mikrovesikeln mit den Schritten: Gewinnen einer ersten Population von Stammzellen, die nicht von einem menschlichen Embryo gewonnen worden sind, von einem Versuchsobjekt; b) Induzieren einer Differenzierung in eine erste Population von Stammzellen, die nicht von einem menschlichen Embryo gewonnen worden sind, indem ein Induktor auf diese Zellen aufgebracht wird, der nicht autogen ist, und c) Ernten von daraus erzeugten Mikrovesikeln, die im Wesentlichen frei von dem Induktor aus Schritt a) sind, wobei die erste Zellpopulation gegenüber den Mikrovesikeln autogen ist.

3. Verfahren gemäß Anspruch 1 oder 2, wobei die erste Zellpopulation aus menschlichen Zellen besteht.

4. Verfahren gemäß einem vorhergehenden Anspruch, wobei die erste Zellpopulation mesenchymale Stammzellen umfasst.

5. Verfahren gemäß einem vorhergehenden Anspruch, wobei die Stammzellen von Knochenmark stammende Stammzellen sind.

6. Verfahren gemäß einem vorhergehenden Anspruch, wobei die Erzeugung von Mikrovesikeln durch die erste Zellpopulation durch eine der nachfolgenden Methoden verbessert wird:
• Belasten der ersten Zellpopulation
• Hitzeschock-Behandeln der ersten Zellpopulation
• Abtöten der ersten Zellpopulation
• Ändern der Temperatur, der CO₂-Konzentration, der O₂-Konzentration, der Zusammensetzung des Mediums oder der Salzgehalte, welchen die erste Zellpopulation ausgesetzt wird.

7. Verfahren zur Erzeugung eines lebenden Zellproduktes aus durch Mikrovesikel vermittelter Zellumwandlung mit den Schritten: a) Gewinnen einer ersten Population von Stammzellen, die nicht von einem menschlichen Embryo gewonnen worden sind, von einem Versuchsobjekt; b) Induzieren einer Differenzierung in diese erste Zellpopulation, indem ein Induktor auf die Zellen aufgebracht wird, der nicht autogen ist, c) Ernten von daraus erzeugten Mikrovesikeln, die frei von dem Induktor aus Schritt b) sind; d) Gewinnen einer zweiten Population von Stammzellen, die nicht von einem menschlichen Embryo gewonnen worden sind, von dem Versuchsobjekt, die autogen gegenüber der ersten Zellpopulation ist; e) Induzieren einer Differenzierung in die zweite Zellpopulation, indem die Mikrovesikel aus Schritt c) auf die zweite Zellpopulation aufgebracht werden; und f) Spalten der umgewandelten Zellen aus Schritt e), um ein autogenes Lebendzellprodukt zu erzeugen, wobei die zweite Zellpopulation induziert wird, um sich durch den Kontakt mit entsprechenden autogenen Mikrovesikeln zu differenzieren, und wobei der Kontakt mit dem Induktor aus Schritt a) vermieden wird.

8. Verfahren gemäß Anspruch 1, weiterhin mit dem Schritt des Konservierens der ersten Zellpopulation nach Schritt a) oder des Konservierens der Mikrovesikel nach Schritt c).

9. Verfahren gemäß Anspruch 2, weiterhin mit dem Schritt des Konservierens der ersten Zellpopulation nach Schritt a) oder des Konservierens der Mikrovesikel nach Schritt c).

## Revendications

1. Procédé de production in vitro d'une population de cellules différenciées comprenant les étapes consistant à : a) obtenir une première population de cellules souches qui n'ont pas été obtenues à partir d'un embryon humain d'un sujet, b) induire une différenciation dans ladite première population de cellules en appliquant un inducteur auxdites cellules qui n'est pas autologue, c) récolter des microvésicules produites à partir de celles-ci qui sont dépourvues de l'inducteur de l'étape b), d) obtenir une deuxième population de cellules souches qui n'ont pas été obtenues à partir d'un embryon humain dudit sujet qui est autologue pour ladite première population de cellules, et e) induire une différenciation dans ladite deuxième population de cellules en appliquant lesdites microvésicules de ladite étape c) à ladite deuxième population de cellules, grâce à quoi la deuxième population de cellules est induite pour se différencier par contact avec des microvésicules correspondantes autologues et grâce à quoi un contact avec l'inducteur de l'étape a) est évité.

2. Procédé de production in vitro de microvésicules comprenant les étapes consistant à : obtenir une première population de cellules souches qui n'ont pas été obtenues à partir d'un embryon humain sujet, b) induire une différenciation dans une première population de cellules souches qui n'ont pas été obtenues à partir d'un embryon humain en appliquant un inducteur auxdites cellules qui n'est pas autologue, et c) récolter des microvésicules produites à partir de celles-ci qui sont globalement dépourvues de l'inducteur de l'étape a), dans lequel ladite première population de cellules est autologue pour lesdites microvésicules.

3. Procédé selon la revendication 1 ou 2, dans lequel ladite première population de cellules sont des cellules humaines.

4. Procédé selon l'une quelconque des revendications précédentes, dans lequel ladite première population de cellules comprend des cellules souches mésenchymateuses.

5. Procédé selon l'une quelconque des revendications précédentes, dans lequel lesdites cellules souches sont des cellules souches issues de la moelle osseuse.

6. Procédé selon l'une quelconque des revendications précédentes, dans lequel la production de microvésicules par ladite première population de cellules est améliorée par l'une des techniques suivantes :
- stress de ladite première population de cellules
- application d'un choc thermique à ladite première population de cellules
- destruction de ladite première population de cellules
- modification de la température, de la concentration en CO₂, de la composition du milieu ou des niveaux de salinité auxquels ladite première population de cellules est exposée.

7. Procédé de génération d'un produit de cellules vivantes à partir d'une transformation de cellules médiées de microvésicules comprenant les étapes consistant à : a) obtenir une première population de cellules souches qui n'ont pas été obtenues à partir d'un embryon humain d'un sujet, b) induire une différenciation dans ladite première population de cellules en appliquant un inducteur auxdites cellules qui n'est pas autologue, c) récolter des microvésicules produites à partir de celles-ci qui sont dépourvues de l'inducteur de l'étape b), d) obtenir une deuxième population de cellules souches qui n'ont pas été obtenues à partir d'un embryon humain dudit sujet qui est autologue pour ladite première population de cellules, e) induire une différenciation dans ladite deuxième population de cellules en appliquant lesdites microvésicules de l'étape c) à ladite deuxième population de cellules, et f) perturber les cellules transformées de l'étape e) pour fournir un produit de cellules vivantes grâce à quoi la deuxième population de cellules est induite pour se différencier par contact avec des microvésicules correspondantes autologues et grâce à quoi un contact avec l'inducteur de l'étape a) est évité.

8. Procédé selon la revendication 1, comprenant en outre l'étape consistant à accumuler la première population de cellules après l'étape a), ou accumuler les microvésicules après l'étape c).

9. Procédé selon la revendication 2, comprenant en outre l'étape consistant à accumuler la première population de cellules après l'étape a), ou accumuler les microvésicules après l'étape c).
